Europäisches Patentamt

European Patent Office

Office européen des brevets

(19)

(11) EP 0 839 534 B1

(12) **EUROPEAN PATENT SPECIFICATION**

(45) Date of publication and mention
of the grant of the patent:
**21.08.2002 Bulletin 2002/34**

(21) Application number: **97920756.0**

(22) Date of filing: **13.05.1997**

(51) Int Cl.⁷: **A61K 31/7004**, A61K 31/198
// (A61K31/7004, 31:198,
A61P39:06)

(86) International application number:
**PCT/ES97/00122**

(87) International publication number:
**WO 97/042961 (20.11.1997 Gazette 1997/50)**

(54) **PHARMACOLOGICAL ASSOCIATION BETWEEN N-ACETYLCYSTEIN AND LEVULOSE FOR PREVENTING CELLULAR DEATH AND RELATED DISEASES**

PHARMAKOLOGISCHE KOMBINATION AUS N-ACETYLCYSTEIN UND LEVULOSE ZUR VERHÜTUNG DES ZELLTODS UND VERWANDTER ERKRANKUNGEN

ASSOCIATION PHARMACOLOGIQUE ENTRE N-ACETYLECYSTEINE ET LEVULOSE POUR LA PREVENTION DE LA MORT CELLULAIRE ET LES MALADIES ASSOCIEES

(84) Designated Contracting States:
**AT BE CH DE DK ES FI FR GB GR IE IT LI LU NL PT SE**

(30) Priority: **16.05.1996 ES 9601091**

(43) Date of publication of application:
**06.05.1998 Bulletin 1998/19**

(73) Proprietor: **Zambon, S.A.**
**08130 Santa Perpetua de Mogoda (ES)**

(72) Inventors:
• **ESTERAS, Antonio**
**E-08015 Barcelona (ES)**
• **BRUSEGUINI, Leonida**
**E-08034 Barcelona (ES)**
• **ESTRELA, José Maria**
**E-46009 Valencia (ES)**

(74) Representative: **Isern Jara, Jaime**
**J. Isern Patentes y Marcas, S.L.**
**Avenida Diagonal no. 463, bis 2**
**008036 Barcelona (ES)**

(56) References cited:
**EP-A- 0 339 508**          **WO-A-93/07857**
**WO-A-94/02036**          **US-A- 4 434 160**

• **K. RITTER: "Untersuchungen über den radioprotektiven Effekt der Sulfhydrylverbindungen N-Acetyl-homocystein-thiolacton und Cystein in Kombination mit Fructose" STRAHLENTHERAPIE, vol. 142, num. 1, 1971, páginas 96-104, XP002041256**

Remarks:
The file contains technical information submitted after the application was filed and not included in this specification

**Description**

The invention makes use of N-acetylcysteine and levulose as cellular protectors in the pharmacological therapy of many diseases that imply cellular death associated to oxidative stress phenomena. The exhaustive enumeration of all pathologies in which the use of the association of N-acetylcysteine and levulose could be suitable would be very long. For this reason, they are grouped in wide categories, only some of them being referred for its special clinical relevance:

1.- Otorrhinolaryngology: Otitis, sinusitis, rhinopharyngitis, laryngotracheitis.
2.- Respiratory System: Acute and chronic bronchitis, bronchial asthma, emphysema, bronchopneumonia, pulmonary tuberculosis.
3.- Hepatology: Acute and chronic hepatophaties.
4.- Cardiovascular System: Myocardial infarction, arteriosclerosis, phlebitis, arteritis.
5.- Renal System: nephritis and nephrosis.
6.- Nervous System: neurodegenerative diseases such as Alzheimer or Parkinson.
7.- Immunology: As immunostimulant in immunodeficiencies such as AIDS or in the immunotherapy of cancer.
8.- Oncology: As anticarcinogenic and as a protector of skeletal muscle cells against the muscle mass loss associated to tumoral cachexia.
9.- Reproductive System: In masculine infertility caused by affectation of spermatozoids (oligospermia, decrease of mobility).
10.- Ophthalmology: Retinitis.
11.- Dermatology: Degenerative dermatitis (i.e. contact dermatitis, medicamentosa dermatitis), affectations of hair follicles (i.e. alopecia).
12.- Nuclear Medicine: As radioprotector against cell lesions derived of exposure to toxic doses of ionizing radiation.

Associations of levulose and N-acetylcysteine (NAC) are already well known in the art. A calorific nutrient solution for human parenteral feeding containing at least one reducing sugar namely glucose and/or fructose and NAC has indeed been described (WO 93 07857). This solution contains 0.01-50 g/l (and preferably 0.05-5 g/l) of NAC together with a 2.5-70% sugar solution. In the same manner, nutritional supplements containing notably fructose and NAC or a mixture of amino acids, from which NAC, together with fructose and L-carnitine have been described (WO 94 02036 and US 4 434 160 respectively). These compositions are meant to be used by humans who have been subjected to physiological stress that accompanies for example radiation therapy or by patients in the post-aggression phase. A mouth-soluble pharmaceutical composition containing notably NAC and fructose has also been described (EP 0 339 508).

Several groups of scientists have shown recently that high concentrations of fructose prevent the anoxic death of hepatic cells. Different mechanisms have been proposed to explain this protective effect:

a) an increase in the glycolytic production of lactate, which can protect directly the cells; b) an increase in the anaerobic synthesis of adenosine triphosphate (ATP); c) the inhibition of the production of free radicals; d) the modulation of $Ca^{2+}$ homeostasis. In spite of these hypothesis, however, the molecular mechanisms involved in the protective effect caused by fructose are not yet elucidated.

Under anaerobic conditions, levulose is a better glycolytic substrate than glucose or other carbohydrates, producing high proportions of lactate. None the less, lactate (10 mM) alone does not prevent cell death by anoxia. Thus, even though lactate can contribute to the low intracellular pH (pHi) found after 30 minutes of anoxia in the presence of levulose, it does not explain by itself the protective role of levulose against anoxic damage. Anoxic damage is clearly reduced by lowering extracellular pH and, consequently, bringing about an intracellular acidification. However, when pHi is ligated to an external pH of 7.4 by the use of monensine, which modulates the exchange of $Na^+$ by $H^+$, levulose still protects against cell death. Therefore, acidosis and levulose can protect against cell death by independent mechanisms.

In aerobic metabolisms the energy required to maintain cell integrity is supplied by the mitochondrial cytochrome system. In anoxia, the lack of oxygen does not allow the formation of ATP by oxidative phosphorylation, and the stored ATP is quickly consumed. Truly, the exhaustion of ATP is a certain fact under conditions of hypoxia or toxic damages. However, the pathway by which a deficit in ATP becomes an irreversible damage is not clear. Levulose has been shown to be effective in the protection of rat hepatocytes against lethal damage caused by mitochondrial inhibitors, such as cyanide, oligomicyn or menadione. Some authors have proposed that the protective mechanism by levulose should involve the glycolytic production of ATP. However, levulose can not protect cells against hypoxic damages by simply maintaining the glycolytic production of ATP.

Liver necrosis is normally accompanied by an increase in the concentration of free intracellular $Ca^{2+}$, which can lead to the activation of the $Ca^{2+}$ dependent degradative enzymes, such as phospholipase A, endonucleases and proteases. The role of free intracellular $Ca^{2+}$ in cytotoxicity has been studied specially in hepatocytes. Glutathione (GSH) plays a critical role in the regulation of $Ca^{2+}$ sequestration in the endoplasmic reticulum. Furthermore, under conditions of increase of glutathione disulfide (GSSG), for instance during oxidative stress, GSSG is reduced to GSH by glutathione reductase, at the expense of nicotinamide-adenosin dinucleotide phosphate (NADPH). Therefore, oxidation of GSH can favor an alteration in mitochondrial $Ca^{2+}$.

Glutathione is always present in eukaryotic cells, being involved in many cellular functions. It is the most prevalent cellular thiol and the most abundant low molecular weight peptide present in cells. GSH acts as reducing agent and as antioxidizer, it serves as a reserve of cysteine, it participates in reactions of xenobiotic detoxification and in the metabolism of numerous cellular compounds, it is required for the synthesis of some prostaglandins, and it is related to the regulation of the cell cycle and to thermotolerance. This tripeptide plays a role in the protection against tissue damages resulting of exposure to oxidizing ambiances such as hyperoxia, hyperbaric oxygen or ozone. It can also protect against radiation, ultraviolet light and photodynamic effects.

As a consequence of all the aforementioned, a possible explanation for the protective effect of levulose against hepatic cell death, under toxic stress conditions, can be resumed in the hypothesis that there can be a relationship between the maintenance of cellular NADPH levels, glutathione status and calcium homeostasis and the incidence of levulose in these cellular phenomena.

On the other hand, several studies have shown the symptomatic improvement that is obtained in some diseases, such as chronic bronchitis, after treatment with N-acetylcysteine (NAC). In spite of some variations between different studies in the effectiveness obtained, there are many data that suggest that NAC can improve the symptoms of chronic bronchitis. The most important improvements reported concern to the decrease in amount and purulence of the sputum, to the rate of exacerbation, and/or the number of days of illness. Among the well-known features of chronic bronchitis are the following: ciliary paralysis, hyperplasia of mucous cells, obstruction by mucus, infection, inflammation and cell lesion (fibrosis). Bronchitis is initiated in many cases by cigarette smoke. As a result of this lesion, a vicious circle is developed, leading to hypoxia, emphysema and, often to death. From a clinical perspective, there are already data that indicate that NAC can diminish the weakener symptoms in patients that suffer from chronic bronchitis.

To explain which can be the mechanisms responsible of the symptomatic improvement of patients with chronic bronchitis treated with NAC, one has to take into account: on one hand, it is probable that the direct mucolytic properties of NAC contribute to the clinical improvement; on the other hand, however, it can also be the case that some of the antioxidizer properties of NAC are beneficial as well to the chronic bronchitis patients. The later assumption is based mainly in the finding that iodoglycerol, a mucolytic that does not possess antioxidizer properties, while reducing effectively some of the symptoms of bronchitis, does not seem to be as efficient clinically as NAC, which is a mucolytic that does have antioxidizer properties.

Among the oxidative mechanisms that can contribute to chronic bronchitis, it can be emphasized that, for example, tobacco smoke can alter the equilibrium oxidizers/antioxidizers in lung, and therefore influence the development of chronic bronchitis in several ways. Among the various possibilities that can produce alterations in the equilibrium oxidizers/antioxidizers are: the increase in the production or conversion of xanthine dehydrogenase (XD) to xanthine oxidase (XO), which leads to an increase in the formation of oxygen metabolites in lung cells. The increase in oxidative stress can, in turn, increase the conversion of GSH to oxidized GSH (GSSG), inactivate the antiproteinases and/or promote cellular hyperplasia. In the same way, tobacco smoke could increase the number and activity of alveolar macrophages or neutrophiles in lung, and augment the release of oxygen radicals by this cells. Moreover, oxygen radicals can form chemotactines that can in turn attract more neutrophiles. The attracted phagocytes could release more oxidizers, as well as elastase, contributing even more to upset, in a synergetic way, the equilibrium oxidizers/antioxidizers and cause cell lesion.

Various studies carried out by different authors to study the effect of NAC on the equilibrium oxidizers/antioxidizers have shown that:

- Alveolar Macrophages (AM) obtained by washing of lungs of asymptomatic cigarette smokers produce more superoxide anion in vitro than AM from lungs of control healthy subjects.
- The addition of neutrophiles stimulated with phorbol myristate acetate (PMA) produced lesion of lung epithelial cells cultivated in vitro, which was reflected by the release of $Cr^{51}$ previously incorporated to the epithelial cells. Treatment with NAC protected substantially against the neutrophile mediated lesion.
- The release of superoxide anion by AM from cigarette smokers is reduced in individuals that have had NAC during 8 weeks.
- The number of cells in the upper and lower respiratory tract in rats exposed to cigarette smoke is reduced by the treatment with NAC.

It conclusion, it can be affirmed that the disequilibrium oxidizers/antioxidizers is a key fact in many diseases, among them chronic bronchitis. A summary of the different factors that cause oxidative lesions, and those that provide antioxidizer defenses can be the following:

Factors that cause oxidative lesions:

- XO
- Phagocytes
- Mitochondria
- AA Metabolism
- Air pollution
- Tobacco

Antioxidizer defenses:

- SOD (superoxide dismutase)
- Catalase
- Vitamin E
- Ceruloplasmin
- Redox reactions of glutathione

NAC can have the capability to shift the equilibrium to favor the antioxidizers, possibly by acting as an efficient agonist of GSH.

Having reviewed, in all of the above described, the advantageous effects for cell maintenance displayed by levulose and N-acetylcysteine separately, we have recently proven, and this is the basis of the present invention, that the association of levulose and N-acetylcysteine shows a strong synergetic protective effect against oxidative stress in isolated rat hepatocytes.

It has been proven that the association of levulose and NAC protects hepatic cells against cellular death induced by tert-butyl-hydroperoxide (tbuOOH). None of these products, when administered alone, can get the high level of cellular protection reached by the association described in the invention. Neither of other known hepatoprotectors, such as methionine, S-adenosylmethionine or arginine-thiazolidin-carboxylate, protect hepatic cells against damage induced by strong oxidizers.

Considering all of the explained above, in one aspect, the present invention includes a pharmacological association that protects cells against different toxic substances or stress conditions,when the proportion of administration of levulose and N-acetylcysteine is comprised between 1/1 and 5/1 molar ratio.

It is an aspect of the invention the association of N-acetylcysteine and levulose as a protector against damages caused by hypoxia/anoxia, oxidative stress, different xenobiotics and ionizing radiation.

A further aspect of the invention is the use of the association of NAC and levulose to preserve the mitochondrial cell functions (membrane potential, oxidative phosphorylation, etc.).

Yet another aspect of the invention is the use of the association of NAC and levulose to prepare a pharmaceutical composition which protects cells against different toxic substances or stress conditions.

Finally, the invention covers a pharmaceutical composition that has an association of NAC and levulose as the active component.

For therapeutical application, the association NAC-levulose is preferentially incorporated to a pharmaceutical composition suitable for oral or parenteral administration.

Depending on the selected mode of administration, the compositions according to the invention can be either in the form of tablets, capsules, pills and the like, or as an easy to use liquid preparation or to be prepared by dilution in the moment of use.

The compositions can contain the association NAC-levulose together with pharmaceutically acceptable carriers, which can be solids or liquids, organic or inorganic pharmaceutical excipients such as disintegrating agents, diluents and the like.

In addition to excipients, compositions can contain preservatives, stabilizers, moisturizers, buffers, colorants and flavors.

As an example, Table 1 shows the protective effect of the association NAC+levulose against oxidative stress in isolated rat hepatocytes.

Table 1 :

| Additions | Cellular Viability (%) | GSH (µmol/g) | GSSG (nmol/g) |
|---|---|---|---|
| None | 83 ± 5 | 3.2 ± 0.3 | 221 ± 68 |
| Glucose | 90 ± 2 | 3.5 ± 0.3 | 25 ± 11 |
| Levulose | 91 ± 4 | 3.6 ± 0.4 | 19 ± 10 |
| NAC | 90 ± 2 | 4.6 ± 0.3 | 29 ± 7 |
| Glucose + NAC | 90 ± 5 | 4.7 ± 0.5 | 31 ± 9 |
| Levulose + NAC | 92 ± 3 | 5.2 ± 0.3 | 18 ± 5 |
| tbuOOH | 0 ± 0 | 0 ± 0 | 2978 ± 58 |
| tbuOOH + glucose | 0 ± 0 | 0.1 ± 0.1 | 2869 ± 101 |
| tbuOOH + levulose | 80 ± 7 | 1.6 ± 0.4 | 1739 ± 259 |
| tbuOOH + NAC | 0 ± 0 | 0.3 ± 0.1 | 2733 ± 185 |
| tbuOOH + glucose + NAC | 0 ± 0 | 0.4 ± 0.1 | 2671 ± 226 |
| tbuOOH +levulose + NAC | 92 ± 3 | 4.0 ± 0.4 | 96 ± 3 |

Isolated hepatocytes were obtained from Wistar rats (200-250 g; fasted for 24 hours) according to the method of Barry & Friend (J. Cell. Biol.,43; 506-520, 1969). Cells were incubated (10-12 mg of dry weight/ml) at 37°C in Krebs-Henseleit (pH 7.4) containing 1.3 mM $CaCl_2$. Gas atmosphere was 95% $O_2$ - 5% $CO_2$. The different substrates were incubated in the following concentrations: glucose, 5 mM; levulose, 2 mM; NAC, 0.5 mM; tert-butylhydroperoxide (tbuOOH), 1 mM. Incubation time was 60 min. Control values at "zero time" are the following: cellular viability (%): 92 ± 2; GSH (µmol/g): 3.5 ± 0.3; GSSG (nmol/g): 52 ± 13.

As demonstrated by the obtained results, the association of NAC and levulose, with simultaneous application, has a very potent antioxidizing effect. Cells exposed to the action of a high concentration of tert-butylhydroperoxide (1 mM) die, by cellular necrosis, in a period shorter than 30 minutes. The time of incubation in the assays of Table 1 is 60 minutes, to allow the observation of the effects at longer periods.

The results indicate that the association of NAC (0.5 mM) and levulose (2 mM) prevents completely cell death by oxidation. None of the two molecules, separately, is able to avoid cellular stress, as shown by the GSSG values, which are an excellent index of oxidative stress.

Levulose, in the absence of NAC, reduces significantly oxidation of GSH and prevents, at short times, cellular necrosis. However, the association of NAC + levulose is necessary to provide a total protection to the cells, either by using the cited ratio levulose/NAC = 2/0.5, or the claimed ratio range to $\frac{1}{1}$ to $\frac{5}{1}$.

The effect observed when incubating with levulose alone is probably due to NADPH synthesis, which is the cofactor involved in GSSG reduction by glutathione reductase. None the less, the partial effect of levulose does not avoid the cumulative damage caused by a sustained oxidative stress. Therefore, cellular viability decreases progressively, until it falls sharply when the levels of GSH are very low. NAC, as an ideal precursor for the synthesis of GSH, rises tripeptide contents to physiological levels.

It is very important to bear in mind that the parameter "cellular viability" (in Table 1) indicates only whether a cell is dead or alive, but not the existence of molecular damages accumulated by the exposure to oxidative stress. This molecular damages diminish metabolic functions of the cells at medium and long term. From that it follows that the redox state of glutathione, which is indeed indicative of cellular damage by oxidation, evidences more clearly the synergetic protective effect of the association NAC-levulose.

The invention covered by this patent can be industrially used in the production and commercialization of pharmaceutical specialties. The following are examples:

AMPOULES

Medicinal substance

- Acetylcysteine        300.0 mg
- Levulose        600.0 mg

Excipient(s)

- Sodium edetate        3.00 mg
- Sodium hydroxide      73.00 mg
- Water for injection        3.00 ml

INJECTION SOLUTION FOR PERFUSION

Active Principles

- Acetylcysteine        2.00 g
- Levulose      4.00 g

Excipients

- Disodium edetate        0.02 g
- Sodium hydroxide q.s.to pH=        6.50
- Water for injection, q.s. to        10.0 ml

BAGS

Medicinal substance

- Acetylcysteine        0.200 g
- Levulose        0.400 g

Excipient(s)

- Orange granulate        0.500 g
- Saccharine        0.008 g
- Orange aroma        0.050 g
- Sunset yellow colorant        0.001 g
- (E-110)
- Saccharose q.s. to        5.000 g

BAGS FOR CHILDREN

Medicinal substance

- Acetylcysteine        0.100 g
- Levulose        0.200 g

Excipient(s)

- Orange granulate          0.500 g
- Saccharine          0.008 g
- Orange aroma          0.050 g
- Sunset yellow colorant          0.001 g
- (E-110)
- Saccharose q.s. to          5.000 g

EFFERVESCENT TABLETS

Active Principle

- Acetylcysteine          600 mg
- Levulose          1200 mg

Excipients

- Sodium bicarbonate          500 mg
- Citric acid          680 mg
- Aspartame          20 mg
- Lemon aroma          100 mg

In association with antibiotics, the synergism of NAC and levulose can be used in the therapy of infectious diseases. The following are non-limiting examples:

CAPSULES

Active Principles

- Thiamphenicol levogire          250.0 mg
- Acetylcysteine          113.0 mg
- Levulose          226.0 mg

Excipients

- Talcum powder          2.5 mg
- Magnesium estearate          2.5 mg

LYOPHILIZED VIALS

Medicinal substance

- Thiamphenicol glycinate acetylcysteinate          405 mg
- (equivalent to thiamphenicol and          250 mg
- Acetylcysteine          113 mg

- Levulose        226 mg

Excipient(s) (per diluent ampoule)

- Disodium edetate        1.25 mg
- Water for injection q.s. to        2.00 ml

LYOPHILIZED VIAL STRONG

Medicinal substance

- Thiamphenicol glycinate acetylcysteinate        1.215 mg
- (equivalent to thiamfenicol and        750 mg
- Acetylcysteine        339 mg
- Levulose        678 mg

Excipient(s) (per diluent ampoule)

- Disodium edetate        3.75 mg
- Water for injection q.s. to        400 mg

BAGS

Active principles

- Amoxicillin trihydrate        287.0 mg
- (equiv. to Amoxicillin anhydrous)        250.0 mg
- N-acetylcysteine        100.0 mg
- Levulose        200.0 mg

Excipients

- Sodium carboxymethylcellulose        50.0 mg
- Orange aroma        100.0 mg
- Acid saccharine        4.0 mg
- Colorant E 110 (Orange A-1)        0.8 mg
- Ethylcellulose        11.6 mg
- Saccharose q.s. to        5 g

BAGS

Active principles

- Amoxicillin trihydrate        574.000 mg
- microencapsulated acetylcysteine        222.200 mg
- Levulose        444.400 mg

Excipients

- Orange aroma        100.000 mg
- Carboxymethylcellulose        50.000 mg
- Acid saccharine        8.000 mg
- Colorant orange A-1        0.800 mg
- Sieved saccharose        4,045.000 mg

**Claims**

1.  A pharmacological association of N-acetylcysteine (NAC) and levulose for the prevention of cellular death, **characterized in that** when both compounds are applied simultaneously, in a proportion levulose/NAC comprised between 1/1 and 5/1 molar ratio, a synergetic cell protective action is displayed, preventing cellular damages caused by various agents.

2.  A pharmacological association according to claim 1 **characterized in that** it produces a clear synergetic effect which provides a strong cell antioxidizing action.

3.  The use of the pharmacological association according to the previous claims for the preparation of pharmaceutical compositions that protect cells against damages induced by hypoxia/anoxia, oxidative stress, different xenobiotic agents, ionizing radiation and other conditions that cause toxic stress.

4.  A pharmaceutical composition according to claim 1 **characterized by** having as an active component an association of NAC and levulose

**Patentansprüche**

1.  Eine pharmakologische Assoziation von N-Acetylcystein (NAC) und Lävulose um Zellentod zu behindern, **dadurch gekennzeichnet dass**, wenn beide Komponenten gleichzeitig angewendet werden, im einem Verhältnis Lävulose/NAC zwischen 1/1 und 5/1 molar, wird eine synergetische schützende Zellwirkung entwickelt, die Zellstörungen verursacht durch verschiedene Mittel vermeidet.

2.  Eine pharmakologische Assoziation nach Anspruch 1, **dadurch gekennzeichnet, dass** eine klare synergetische Wirkung erzeugt, welche eine starke oxydationsverhindernde Zellenwirkung ergibt.

3.  Die Verwendung der pharmakologischen Assoziation nach den vorhergehenden Ansprüchen, für die Herstellung von pharmakologischen Verbindungen, die Zellen schutzen gegen Schäden verursacht durch hypoxia/anoxia, Oxydationstress, verschiedene xenobiotic Mitteln, ionisierende Strählung und andere Bedingungen die giftigen Sress verursachen

4.  Eine pharmakologische Assoziation nach Anspruch 1, die **gekennzeichnet ist, durch** eine Assoziation von NAC und Lävulose als eine wirksam Komponent hat.

**Revendications**

1.  Une association pharmacologique de la N-acetylcystéine (NAC) et la levulose pour la prévention de la mort cellulaire, caractérisée en ce que lorsque les deux composés sont appliqués simultanément, dans une proportion levulose/NAC au rapport molaire compris entre 1/1 et 5/1, une action synergique protectrice des cellules apparait, prévenant des dommages cellulaires produits par divers agents.

2.  Une association pharmacologique selon la revendication 1, caractérisée en cé qu'elle produit un clair effet synergique entraînant une forte action antioxydante des cellules.

3. L'usage de l'association pharmacologique selon les revendications précédentes, pour la préparation de compositions pharmaceutiques protégeant les cellules contre les dommages dérivés de l'hipoxie/anoxie, de l'stress oxydative, de divers agents xenobiotiques, des radiations ionisantes et d'autres conditions causant d'stress toxique.

4. Une composition pharmaceutique selon la revendication 1, charactérisée en ce qu'elle comprend comme un composant actif une association de la NAC et la levulose.